# EUROPÄISCHE PATENTSCHRIFT

(11) **EP 2 714 707 B1**
(45) Veröffentlichungstag und Bekanntmachung des Hinweises auf die Patenterteilung: **14.12.2016**
(21) Anmeldenummer: 12735774.7
(22) Anmeldetag: 25.05.2012
(51) Int. Cl.: C07H 15/252

(54) **KRISTALLISIERUNG VON EPIRUBICINHYDROCHLORID**
CRYSTALLIZATION OF EPIRUBICIN HYDROCHLORIDE
CRISTALLISATION D'HYDROCHLORURE D'ÉPIRUBICINE

(30) Priorität: 31.05.2011 DE 102011103751; 03.06.2011 US 201161493034 P
(43) Veröffentlichungstag der Anmeldung: 09.04.2014
(73) Patentinhaber: medac Gesellschaft für klinische Spezialpräparate mbH, 22880 Wedel (DE)
(72) Erfinder: KUNNARI, Tero, 63739 Aschaffenburg (DE)
(74) Vertreter: Vossius & Partner Patentanwälte Rechtsanwälte mbB
(86) Internationale Anmeldenummer: PCT/EP2012/002248
(87) Internationale Veröffentlichungsnummer: WO 2012/163508

(56) Entgegenhaltungen:
- EP-A1- 1 036 797
- WO-A1-2010/039159
- WO-A2-2011/118929
- US-B1- 6 747 012
- US-B2- 7 485 707

## Beschreibung

Die vorliegende Erfindung betrifft ein Verfahren zur Herstellung von kristallinem Epirubicinhydrochlorid.

Epirubicin und seine Säureadditionssalze, wie zum Beispiel Epirubicinhydrochlorid, sind Verbindungen aus der Gruppe der Anthracycline, die seit den 1980er Jahren als Zytostatika für die Behandlung verschiedener solider Tumorarten eingesetzt werden. Die Struktur von Epirubicinhydrochlorid kann durch die folgende Formel wiedergegeben werden:

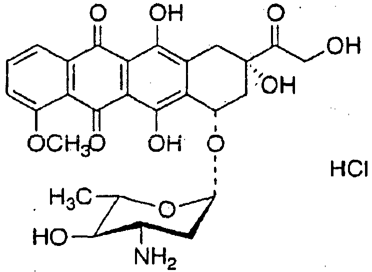

Der Einsatz von Epirubicin zur Behandlung von Tumoren ist zum Beispiel Gegenstand der US 5,091,373.

Die Herstellung von Epirubicin ist unter anderem in den Patenten US 4,112,076 und US 5,874,550 beschrieben. Beispielsweise können Epirubicin und seine Säureadditionssalze ausgehend von Daunorubicin auf chemischem Wege synthetisiert werden. Die Herstellung von Epirubicin durch Fermentation von Mikroorganismen ist jedoch ebenso möglich und zum Beispiel in der EP 1990405 offenbart. Bei der Herstellung von Epirubicin fallen üblicherweise organische und anorganische Verunreinigungen an, deren Anteil bis zu 25 Gewichtsprozent an der hergestellten Produktmischung ausmachen kann. Aus diesem Grund ist eine Aufreinigung von Epirubicin oder seiner entsprechenden Säureadditionssalze nach der Herstellung unabdingbar.

Ein geeignetes Verfahren zur Aufreinigung von Epirubicinhydrochlorid geht aus der US 4,861,870 hervor. Dabei wird Epirubicinhydrochlorid aus einer wässrigen Lösung mit Hilfe von Aceton präzipitiert und als amorpher Feststoff erhalten. Mit diesem Verfahren gelingt es, amorphes Epirubicinhydrochlorid in weitestgehend reiner Form zu erhalten.

In der US 7,485,707 und der WO 2010/039159 ist beschrieben, dass bestimmte kristalline Formen von Epirubicinhydrochlorid, die durch verschiedene Röntgenbeugungsmuster charakterisiert sind, gegenüber den bekannten Modifikationen von Epirubicinhydrochlorid verbesserte thermische Stabilitäten zeigen. Diese kristallinen Modifikationen sollen erhalten werden können, indem man Epirubicinhydrochlorid aus einer Lösung oder einem Gel durch Zugabe eines hydrophilen organischen Lösungsmittels ausfällt. Bei der Nacharbeitung der in diesen Patentdokumenten beschriebenen Verfahren wurde jedoch festgestellt, dass kristallines Epirubicinhydrochlorid mit den beschriebenen Röntgenbeugungsmustern unter den angegebenen Bedingungen nicht erhalten werden kann.

Weiterhin ist aus dem Stand der Technik das Problem bekannt, dass es bei der Herstellung bzw. der Kristallisation von Epirubicinhydrochlorid zu einer unerwünschten Bildung von Dimeren und von Zersetzungsprodukten, wie Doxorubicinon, kommt.

Es besteht daher weiterhin Bedarf an einer thermisch stabilen Modifikation von kristallinem Epirubicinhydrochlorid und einem einfachen und zuverlässigen Verfahren zur Herstellung einer solchen thermisch stabilen Modifikation von kristallinem Epirubicinhydrochlorid in hoher Reinheit.

Der Erfindung liegt daher die Aufgabe zugrunde, eine thermisch stabile Modifikation von kristalline Epirubicinhydrochlorid zur Verfügung zu stellen.

Ferner liegt der Erfindung die Aufgabe zugrunde, ein einfaches und zuverlässiges Verfahren zur Herstellung einer solchen thermisch stabilen Modifikation von kristallinem Epirubicinhydrochlorid in hoher Reinheit bereitzustellen.

Diese Aufgaben werden gelöst durch die Gegenstände der unabhängigen Ansprüche.

Die Erfindung stellt daher ein Verfahren zur Herstellung von kristallinem Epirubicinhydrochlorid zur Verfügung, umfassend die Schritte
(a) Bereitstellung von Epirubicinhydrochlorid,
(b) Herstellung einer Mischung, die das bereitgestellte Epirubicinhydrochlorid und wenigstens einen Alkohol, der aus der Gruppe ausgewählt ist, die aus 1-Butanol, 2-Butanol und 1-Pentanol besteht, enthält, und
(c) Kristallisierung von Epirubicinhydrochlorid aus dieser Mischung; wobei die Mischung in Schritt (b) ferner Wasser enthält.

Offenbart ist ferner kristallines Epirubicinhydrochlorid, das durch dieses Verfahren erhältlich ist.

Erfindungsgemäß wird kristallines Epirubicinhydrochlorid hergestellt.

Dieses kristalline Epirubinhydrochlorid weist vorzugsweise einen Peak in einem Differential Scanning Calorimetry (DSC)-Diagramm mit einer maximalen Intensität im Temperaturbereich von 195 - 205°C, mehr bevorzugt mit einer maximalen Intensität in einem Temperaturbereich von 198 - 202°C und insbesondere mit einer maximalen Intensität bei einer Temperatur von 200°C auf. Bei diesem Peak handelt es sich vorzugsweise um einen exothermen Peak.

Gemäß einer weiteren bevorzugten Ausführungsform weist das kristalline Epirubicinhydrochlorid, welches durch das erfindungsgemäße Verfahren erhältlich ist, einen weiteren Peak im Differential Scanning Calorimetry
(DSC)-Diagramm mit einer maximalen Intensität im Temperaturbereich von 240 - 260°C und insbesondere mit einer maximalen Intensität im Temperaturbereich von 245 - 255°C auf. Bei diesem weiteren Peak handelt es sich vorzugsweise um einen endothermen Peak.

Das Differential Scanning Calorimetry (DSC)-Diagramm kann im Rahmen der Erfindung beispielsweise erhalten werden durch das Aufheizen einer Probe des kristallinen Epirubicinhydrochlorids (zum Beispiel entsprechend einer Menge von 1 - 8 mg Epirubicinhydrochlorid) auf 30 bis 350°C mit einer Heizrate von 10 - 20 K/min, vorzugsweise von 10 K/min, in einem DSC-Kalorimeter.

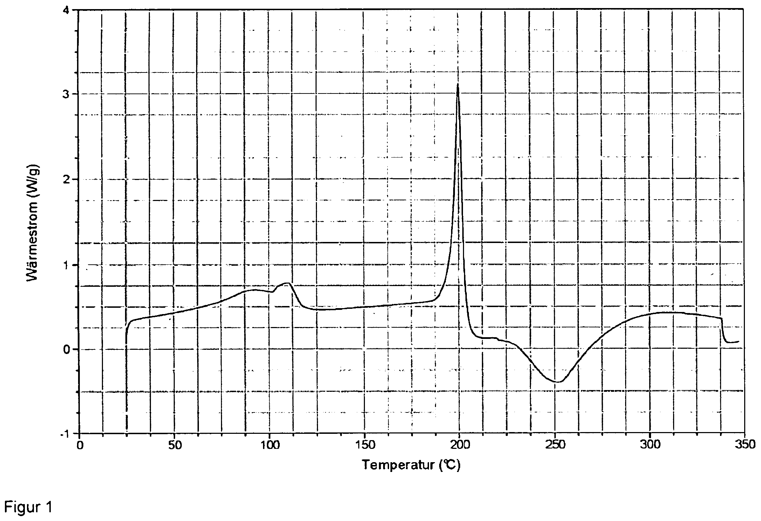

Ein typisches DSC-Diagramm des kristallinen Epirubicinhydrochlorids , welches durch das erfindungsgemäße Verfahren erhältlich ist, ist in FIGUR 1 gezeigt.

Das kristalline Epirubicinhydrochlorid, welches durch das erfindungsgemäße Verfahren erhältlich ist, ist vorzugsweise wenigstens durch Peaks in

einem Pulverröntgenbeugungsdiagramm bei mittleren Werten für den Beugungswinkel (20) in den folgenden Bereichen gekennzeichnet: 5,04- 5,14, 9,00 - 9,20, 13,50- 13,80, 22,00-22,20, 22,40 - 22,50, 22,51 - 22,60, 23,90 - 24,10 und 25,70 - 25,90.

Gemäß einer bevorzugten Ausführungsform weist das kristalline Epirubicinhydrochlorid wenigstens Peaks bei den folgenden mittleren Werten für den Beugungswinkel (20) in einem Pulverröntgenbeugungsdiagramm auf: 5,09, 9,10, 13,63, 22,10, 22,46, 22,52, 24,00 und 25,77.

Gemäß einer besonders bevorzugten Ausführungsform ist das kristalline Epirubicinhydrochlorid Θdurch ein Pulverröntgenbeugungsmuster mit relativen Intensitäten P(%) bei mittleren Werten für den Beugungswinkel (20) gemäß der nachstehenden Tabelle gekennzeichnet:

| **Beugungswinkel (2Θ)** | **Relative Intensität P(%)** | **Bevorzugte relative Intensität** P(%) |
|---|---|---|
| 5,09 | 100-80 | 100 |
| 9,10 | 80-60 | 71 |
| 13,63 | 100-80 | 98 |
| 22, 10 | 55-45 | 50 |
| 22,46 | 95-75 | 85 |
| 22,52 | 100-80 | 100 |
| 24,00 | 100-80 | 100 |
| 25,77 | 65-50 | 56 |

Gemäß einer weiteren bevorzugten Ausführungsform ist dieses kristalline Epirubinhydrochlorid vorzugsweise wenigstens durch Peaks bei den folgenden mittleren Werten für den Beugungswinkel (2Θ) in einem Pulverröntgenbeugungsdiagramm gekennzeichnet: 5,09, 9,10, 9,47, 11,51, 12,01, 12,34, 13,62, 14,59, 16,11, 16,37, 16,50, 18,02, 19,11, 19,36, 20,82, 21,02, 21,37, 22,10, 22,46, 22,52, 23,29, 24,00, 25,77, 27,67 und 29,69.

Gemäß einer weiteren besonders bevorzugten Ausführungsform ist das kristalline Epirubicinhydrochlorid durch ein Pulverröntgenbeugungsmuster mit relativen Intensitäten P(%) bei mittleren Werten für den Beugungswinkel (2Θ) gemäß der nachstehenden Tabelle gekennzeichnet, wobei nur relative Intensitäten P ≥ 10% angegeben sind:

| **Beugungswinkel (2Θ)** | **Relative Intensität P(%)** | **Bevorzugte relative Intensität P(%)** |
|---|---|---|
| 5,09 | 100-80 | 100 |
| 9,10 | 80-60 | 71 |
| 9,47 | 15-10 | 13 |
| 11,51 | 25-18 | 22 |
| 12,02 | 22-14 | 18 |
| 12,34 | 30-20 | 26 |
| 13,63 | 100-80 | 98 |
| 14,59 | 60-40 | 49 |
| 16,11 | 40-30 | 34 |
| 16, 50 | 45-33 | 37 |
| 18,02 | 30-20 | 24 |
| 19,11 | 25-15 | 21 |
| 19,36 | 35-25 | 29 |
| 20,82 | 25 -15 | 20 |
| 21,02 | 33-20 | 27 |
| 21,37 | 40-50 | 46 |
| 22,10 | 55-45 | 50 |
| 22,46 | 95-75 | 85 |
| 22,52 | 100-80 | 100 |
| 24,00 | 100-80 | 100 |
| 25,77 | 65-50 | 56 |
| 27,67 | 55-40 | 47 |
| 29,69 | 25-40 | 32 |

Es kann bevorzugt sein, dass unter dem Begriff "Peak" das Signal dieses Peaks mit der maximalen Intensität verstanden wird.

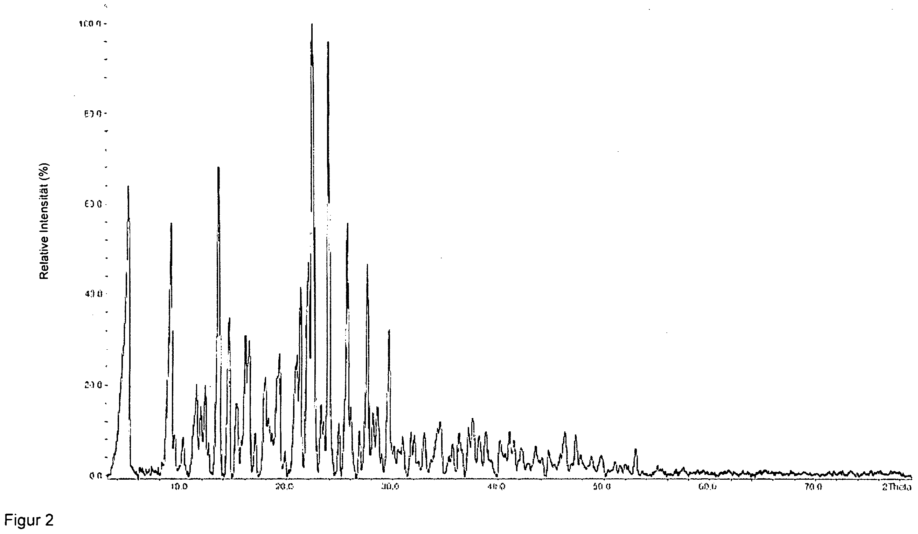

Ein typisches Pulverröntgenbeugungsdiagramm des erfindungsgemäß hergestellten kristallinen Epirubicinhydrochlorids ist in FIGUR 2 gezeigt.

Die vorstehenden Werte beziehen sich auf Röntgenbeugungsmessungen, die mit einem Pulverröntgendiffraktometer der Firma Stoe (Darmstadt) mittels eines IPPSD-Detektors *(image-plate position-sensitive detector*) unter Verwendung von Cu-Kα-Strahlung (A = 1.5406 A) gemessen wurden (Ge-Monochromator). Der Messbereich für 20 war 3 bis 79. Die Messgeräte wurden gegen Si 5N = 99,999 % kalibriert. Die Genauigkeit der erhaltenen Werte beträgt 1,0 %.

Zur Herstellung von kristallinem Epirubicinhydrochlorid wird in einem Schritt (a) zunächst Epirubicinhydrochlorid bereitgestellt.

Dieses Epirubicinhydrochlorid kann auf bekannte Weise, zum Beispiel fermentativ oder synthesechemisch, hergestellt worden sein.

Die Bereitstellung von Epirubicinhydrochlorid kann als Feststoff, in Suspension oder in Lösung erfolgen. Vorzugsweise wird Epirubicinhydrochlorid in fester Form oder in Lösung bereitgestellt.

Wird Epirubicinhydrochlorid als Feststoff bereitgestellt, so kann dieser als amorphes Epirubicinhydrochlorid oder als kristallines Epirubicinhydrochlorid vorliegen.

Wird Epirubicinhydrochlorid in Lösung bereitgestellt, so handelt es sich hierbei um eine wässrige Lösung von Epirubicinhydrochlorid. Gemäß einer besonders bevorzugten Ausführungsform ist diese wässrige Lösung eine konzentrierte wässrige Lösung von Epirubicinhydrochlorid. Unter wässriger Lösung von Epirubicinhydrochlorid wird erfindungsgemäß eine Lösung verstanden, die Epirubicinhydrochlorid und Wasser enthält. Der Anteil von Wasser in dieser Lösung liegt vorzugsweise im Bereich von 30 - 70 Volumenprozent und mehr bevorzugt im Bereich von 40 - 60 Volumenprozent, bezogen auf das Gesamtvolumen der Epirubicinhydrochlorid enthaltenden wässrigen Lösung. Neben Epirubicinhydrochlorid und Wasser kann die wässrige Lösung jedoch gegebenenfalls noch weitere Bestandteile, insbesondere wenigstens ein weiteres Lösungsmittel, enthalten. Bei diesem wenigstens einen weiteren Lösungsmittel kann es sich beispielsweise um einen Alkohol handeln. Als Alkohol sind dabei Ethanol, 1-Propanol, 2-Propanol oder Mischungen davon bevorzugt. Der Anteil an dem wenigstens einen Alkohol liegt vorzugsweise im Bereich von 30 - 70 Volumenprozent und mehr bevorzugt im Bereich von 40 - 60 Volumenprozent, bezogen auf das Gesamtvolumen der Epirubicinhydrochlorid enthaltenden wässrigen Lösung. Der Gehalt an Epirubicinhydrochlorid in dieser wässrigen Lösung beträgt vorzugsweise 100 - 400 g/l und mehr bevorzugt 150 - 350 g/l, bezogen auf das Gesamtvolumen der Epirubicinhydrochlorid enthaltenden wässrigen Lösung. Gemäß einer bevorzugten Ausführungsform liegt der pH-Wert der Epirubicinhydrochlorid enthaltenden wässrigen Lösung im Bereich von 3,5 - 4,5.

Das in Schritt (a) bereitgestellte Epirubicinhydrochlorid wird in einem Schritt (b) zur Herstellung einer Mischung verwendet.

Hierzu wird das bereitgestellte Epirubicinhydrochlorid, das vorzugsweise als Feststoff oder in Lösung vorliegt, mit wenigstens einem Alkohol vereinigt, der aus der Gruppe ausgewählt ist, die aus 1-Butanol, 2-Butanol und 1-Pentanol besteht.

Dementsprechend wird eine Mischung gebildet, die wenigstens Epirubicinhydrochlorid und wenigstens einen Alkohol, der aus der Gruppe ausgewählt ist, die aus 1-Butanol, 2-Butanol und 1-Pentanol besteht, enthält. Als besonders vorteilhaft für die Kristallisation hat es sich erwiesen, eine Mischung herzustellen, die neben Epirubicinhydrochlorid wenigstens 1-Butanol enthält.

Die Gegenwart eines Alkohols, der aus der Gruppe ausgewählt ist, die aus 1-Butanol, 2-Butanol und 1-Pentanol besteht, insbesondere von 1-Butanol, trägt überraschenderweise dazu bei, die für Epirubicinhydrochlorid ansonsten typische Gelbildung, die einer Kristallisierung von Epirubicinhydrochlorid im Wege steht, zu verhindern. Dementsprechend wird erst durch die Gegenwart von wenigstens einem Alkohol, der aus der Gruppe ausgewählt ist, die aus 1-Butanol, 2-Butanol und 1-Pentanol besteht, das Wachstum von Epirubicinhydrochlorid-Kristallen ermöglicht.

Gemäß einer bevorzugten Ausführungsform liegt der Anteil des wenigstens einen Alkohols, der aus der Gruppe ausgewählt ist, die aus 1-Butanol, 2-Butanol und 1-Pentanol besteht, im Bereich von 5 - 100 Volumenprozent, mehr bevorzugt im Bereich von 5 - 50 Volumenprozent, noch mehr bevorzugt im Bereich von 5 - 30 Volumenprozent, besonders bevorzugt im Bereich von 6 - 20 Volumenprozent und ganz besonders bevorzugt im Bereich von 7 - 15 Volumenprozent, bezogen auf das Gesamtvolumen der Mischung in Schritt (b). Bei einer Konzentration von weniger als 5 Volumenprozent an dem wenigstens einen Alkohol, der aus der Gruppe ausgewählt ist, die aus 1-Butanol, 2-Butanol und 1-Pentanol besteht, bezogen auf das Gesamtvolumen der Mischung, hat sich gezeigt, dass die Tendenz zur Kristallisierung von Epirubicinhydrochlorid erheblich abnimmt.

Gemäß einer weiteren bevorzugten Ausführungsform enthält die Mischung in Schritt (b) neben Epirubicinhydrochlorid und wenigstens einem Alkohol, der aus der Gruppe ausgewählt ist, die aus 1-Butanol, 2-Butanol und 1-Pentanol besteht, wenigstens einen weiteren Alkohol.

Dieser weitere Alkohol ist vorzugsweise aus der Gruppe ausgewählt, die aus Ethanol, 1-Propanol und 2-Propanol besteht. Gemäß einer besonders bevorzugten Ausführungsform handelt es sich bei dem weiteren Alkohol um 2-Propanol.

Vorzugsweise liegt der Anteil des wenigstens einen weiteren Alkohols, der aus der Gruppe ausgewählt ist, die aus Ethanol, 1-Propanol und 2-Propanol besteht, im Bereich von 5 - 95 Volumenprozent, mehr bevorzugt im Bereich von 10 - 94 Volumenprozent, noch mehr bevorzugt im Bereich von 50 - 93 Volumenprozent, besonders bevorzugt im Bereich von 75 - 92 Volumenprozent und ganz besonders bevorzugt im Bereich von 80 - 90 Volumenprozent, bezogen auf das Gesamtvolumen der Mischung.

Ist in der Mischung ein weiterer Alkohol enthalten, der aus der Gruppe ausgewählt ist, die aus Ethanol, 1-Propanol und 2-Propanol besteht, so kann es bevorzugt sein, dass das Verhältnis des Volumens dieses weiteren Alkohols zu dem Volumen des Alkohols, der aus der Gruppe ausgewählt ist, die aus 1-Butanol, 2-Butanol und 1-Pentanol besteht, im Bereich von 3 : 1 bis 20 : 1, mehr bevorzugt im Bereich von 5 : 1 bis 15 : 1 und noch mehr bevorzugt im Bereich von 7 : 1 bis 10 : 1 liegt.

Die in Schritt (b) hergestellte Mischung kann zusätzlich noch weitere Bestandteile aufweisen. Vorzugsweise liegt der Anteil an Wasser unter 7 Volumenprozent, bezogen auf das Gesamtvolumen der Mischung. Ein höherer Anteil von Wasser in der Mischung kann eine Reduzierung der Ausbeute zur Folge haben. Gemäß einer bevorzugten Ausführungsform liegt der Anteil an Wasser im Bereich von 0,5 - 7 Volumenprozent und mehr bevorzugt im Bereich von 3 - 5 Volumenprozent, bezogen auf das Gesamtvolumen der Mischung.

Als besonders vorteilhaft hat es sich herausgestellt, wenn in Schritt (b) eine Mischung hergestellt wird, die neben Epirubicinhydrochlorid 80 - 90 Volumenprozent 2-Propanol, 5 - 15 Volumenprozent 1-Butanol und 2 - 6 Volumenprozent Wasser, bezogen auf das Gesamtvolumen der Mischung, enthält.

Erfindungsgemäß kann es ferner vorteilhaft sein, dass der Anteil von Epirubicinhydrochlorid im Bereich von 5 - 100 g/l, vorzugsweise im Bereich von 6 - 100 g/l, mehr bevorzugt im Bereich von 10 - 50 g/l und noch mehr bevorzugt im Bereich von 25 - 35 g/l, bezogen auf das Gesamtvolumen der Mischung in Schritt (b), liegt. Eine Konzentration von Epirubicinhydrochlorid in diesem Bereich führt zu einer überraschend hohen Ausbeute an kristallinem Epirubicinhydrochlorid, die in diesem Fall beispielsweise bei etwa 95 Gewichtsprozent liegen kann.

Die in Schritt (b) hergestellte Mischung kann eine Lösung oder eine Suspension sein. Eine Lösung von Epirubicinhydrochlorid wird üblicherweise erhalten, wenn vor der Zugabe des wenigstens einen Alkohols eine Lösung von Epirubicinhydrochlorid, beispielsweise eine wässrige Lösung von Epirubicinhydrochlorid, vorliegt. Dagegen liegt die Mischung in Schritt (b) üblicherweise als Suspension vor, wenn vor der Zugabe des wenigstens einen Alkohols Epirubicinhydrochlorid als Feststoff vorliegt.

Als besonders vorteilhaft für die Kristallisierung hat sich ein pH-Wert der Mischung in Schritt (b) im Bereich von 2,5 - 4,5 erwiesen. Eine optimale Kristallisierung wird dabei erhalten, wenn der pH-Wert der Mischung in Schritt (b) im Bereich von 3,0 - 4,5, mehr bevorzugt im Bereich von 3,5 - 4,5 und insbesondere im Bereich von 3,9 - 4,1 liegt. Wird die Mischung durch Zugabe des wenigstens einen Alkohols zu Epirubicinhydrochlorid als Feststoff hergestellt, so weist die Mischung üblicherweise bereits einen pH-Wert in diesem Bereich auf. Wenn die Herstellung der Mischung durch Zugabe des wenigstens einen Alkohols zu einer Epirubicinhydrochlorid enthaltenden Lösung erfolgt, so kann die Mischung einen höheren pH-Wert aufweisen. In diesem Fall kann der pH-Wert auf den bevorzugten Bereich zum Beispiel durch Zugabe von Salzsäure eingestellt werden.
In Schritt (c) erfolgt die Kristallisierung von Epirubicinhydrochlorid.

Dazu kann die in Schritt (b) erhaltene Mischung beispielsweise stehen gelassen werden, bis sich kristallines Epirubicinhydrochlorid bildet. Falls erforderlich, kann die Mischung dabei gerührt werden.

Die Mischung kann zur Beschleunigung der Kristallisierung jedoch auch erwärmt werden. Gemäß einer bevorzugten Ausführungsform wird die Mischung auf eine Temperatur im Bereich von 40 - 80°C, mehr bevorzugt im Bereich von 50 - 75°C und noch mehr bevorzugt im Bereich von 60 - 70°C erwärmt. Bei Temperaturen von unter 40°C erfolgt die Kristallisierung von Epirubicinhydrochlorid aus der Mischung nur langsam, während bei Temperaturen von über 80°C das in der Mischung erhaltene Epirubicinhydrochlorid langsam zersetzt wird. Das Erwärmen der Mischung erfolgt vorzugsweise unter Rühren.

Gemäß einer weiteren bevorzugten Ausführungsform wird die Mischung bei einer Temperatur im vorstehend angegebenen Bereich für einen Zeitraum von wenigstens zwei Stunden, beispielsweise für einen Zeitraum im Bereich von 2 - 8 Stunden, 4 - 8 Stunden oder 4 - 6 Stunden, belassen. Auch dabei kann die Mischung gegebenenfalls gerührt werden.

Im Anschluss kann die erwärmte Mischung abgekühlt werden. Das Abkühlen kann beispielsweise auf eine Temperatur im Bereich von 20 - 30°C, insbesondere auf eine Temperatur von 25°C, erfolgen.

Es hat sich herausgestellt, dass kristallines Epirubicinhydrochlorid thermodynamisch stabiler ist als amorphes Epirubicinhydrochlorid. Bei einer Kristallisierung von Epirubicinhydrochlorid aus einer Lösung wird üblicherweise unmittelbar kristallines Epirubicinhydrochlorid erhalten. Erfolgt die Kristallisierung von Epirubicinhydrochlorid aus einer Suspension, die amorphes Epirubicinhydrochlorid enthält, so wird üblicherweise das als Feststoff in der Suspension zunächst vorliegende amorphe Epirubicinhydrochlorid allmählich in das thermodynamisch stabilere kristalline Epirubicinhydrochlorid umgewandelt.

Nach der Kristallisierung können die erhaltenen Kristalle von der übrigen Mischung abgetrennt werden. Das Abtrennen erfolgt dabei vorzugsweise durch Filtrieren oder Destillieren.

Falls erforderlich oder gewünscht, können die Kristalle danach gewaschen werden. Das Waschen kann beispielsweise mit einem Keton, wie zum Beispiel Aceton, erfolgen.

Nach der optionalen Waschung der Kristalle können die Kristalle von der Waschlösung wiederum abgetrennt werden. Auch hier erfolgt das Abtrennen üblicherweise durch Filtration oder Destillation.

Der isolierte Feststoff kann schließlich getrocknet werden. Das Trocknen erfolgt vorzugsweise bis zur Gewichtskonstanz und ebenfalls vorzugsweise unter Vakuum.

Die Erfindung wird nachstehend anhand von Beispielen beschrieben, die jedoch den Schutzbereich nicht beschränken sollen.

### BEISPIELE

### BEISPIEL 1:

9,0 g amorphes Epirubicinhydrochlorid wurden in einer Mischung aus 12 ml Wasser, 258 ml 2-Propanol und 30 ml 1-Butanol suspendiert. Diese Suspension wurde unter Rühren auf 65°C erhitzt und bei dieser Temperatur für vier Stunden belassen. Dabei wurde der in der Suspension enthaltene Feststoff nicht vollständig gelöst, sondern wandelte sich allmählich von einer amorphen Modifikation in eine kristalline Modifikation um. Die Suspension wurde stufenweise auf eine Temperatur von 22°C abgekühlt. Nach der Entfernung der in der Suspension enthaltenen Lösungsmittel durch Filtration wurden die Kristalle mit Aceton gewaschen und nach der Entfernung von Aceton für 24 Stunden im Vakuum getrocknet.

Anschließend wurde die Reinheit des erhaltenen Epirubicinhydrochlorids überprüft. Die Gegenwart von Dimeren oder Zersetzungsprodukten wurde nicht festgestellt. Die Ausbeute betrug 95%.

Das erhaltene kristalline Epirubicinhydrochlorid wurde einem Test auf thermische Stabilität unterzogen. Dazu wurden die erhaltenen Kristalle bei einer Temperatur von 40°C für einen Zeitraum von einer Woche, zwei Wochen bzw. drei Wochen gelagert. Innerhalb dieser Zeitfenster konnte kein Abbau des kristallinen Epirubicinhydrochlorids festgestellt werden. Vielmehr blieben die Kristalle in unveränderter Form erhalten.

### BEISPIEL 2:

10,0 g amorphes Epirubicinhydrochlorid wurden in einer Mischung aus 13 ml Wasser und 13 ml 2-Propanol gelöst, um eine Epirubicinhydrochlorid enthaltende Lösung bereitzustellen. Diese Lösung wurde anschließend mit 33 ml 1-Butanol und 274 ml 2-Propanol versetzt. Die erhaltene Mischung wurde auf 65°C erwärmt und bei dieser Temperatur für vier Stunden stehen gelassen, wobei sich Epirubicinhydrochlorid-Kristalle bildeten. Danach wurde die erhaltene Suspension stufenweise auf eine Temperatur von 22°C abgekühlt. Die in der Suspension enthaltenen Lösungsmittel wurden durch Filtration entfernt und die als Filterrückstand übrig gebliebenen Kristalle mit Aceton gewaschen. Nach der Entfernung des Acetons wurden die Kristalle für 24 Stunden unter Vakuum getrocknet.

Anschließend wurde die Reinheit des erhaltenen Epirubicinhydrochlorids überprüft. Die Gegenwart von Dimeren oder Zersetzungsprodukten wurde nicht festgestellt. Die Ausbeute betrug 95%.

Das erhaltene kristalline Epirubicinhydrochlorid wurde einem Test auf thermische Stabilität unterzogen. Dazu wurden die erhaltenen Kristalle bei einer Temperatur von 40°C für einen Zeitraum von einer Woche, zwei Wochen bzw. drei Wochen gelagert. Innerhalb dieser Zeitfenster konnte kein Abbau des kristallinen Epirubicinhydrochlorids festgestellt werden. Vielmehr blieben die Kristalle in unveränderter Form erhalten.

### VERGLEICHSBEISPIEL 1:

Es wurde eine Lösung von Epirubicinhydrochlorid (10.0 g) in Wasser (pH 3,5) hergestellt, die einer Trocknung unter Vakuum bei einer Temperatur von 40°C unterzogen wurde, bis ein gelähnlicher Zustand erreicht war. Die so erhaltene Lösung wurde mit 300 ml Aceton versetzt, um Epirubicinhydrochlorid aus dieser Lösung zu präzipitieren. Das erhaltene Präzipitat wurde aus der Lösung durch Filtration gewonnen und mit 50 ml Aceton gewaschen.
Anschließend wurde die Reinheit des erhaltenen Epirubicinhydrochlorids überprüft. Die Ausbeute betrug zunächst 95%. Es wurde die Gegenwart von Dimeren festgestellt.

Das erhaltene Epirubicinhydrochlorid wurde einem Test auf thermische Stabilität unterzogen. Dazu wurde das Epirubicinhydrochlorid bei einer Temperatur von 40°C für einen Zeitraum von einer Woche, zwei Wochen bzw. drei Wochen gelagert. Innerhalb dieser Zeitfenster wurde ein sich mit zunehmender Lagerdauer verstärkender thermisch bedingter Abbau von Epirubicinhydrochlorid um jeweils zwei Prozent beobachtet.

### VERGLEICHSBEISPIEL 2:

Es wurde Beispiel 1 der US 7,485,707 gefolgt und zunächst eine Lösung von Epirubicinhydrochlorid (10.0 g) in Wasser (pH 3,5) hergestellt, die einer Trocknung unter Vakuum bei einer Temperatur von 40°C unterzogen wurde, bis ein gelähnlicher Zustand erreicht war. Die so erhaltene Lösung wurde mit dem zwölffachen Volumen an 1-Propanol versetzt und für drei Stunden bei einer Temperatur von 60°C gerührt. Es wurde kein kristallines Epirubicinhydrochlorid gemäß der vorliegenden Erfindung erhalten.

## Patentansprüche

1. Verfahren zur Herstellung von kristallinem Epirubicinhydrochlorid, umfassend die Schritte
(a) Bereitstellung von Epirubicinhydrochlorid,
(b) Herstellung einer Mischung, die das bereitgestellte Epirubicinhydrochlorid und wenigstens einen Alkohol, der aus der Gruppe ausgewählt ist, die aus 1-Butanol, 2-Butanol und 1-Pentanol besteht, enthält, und
(c) Kristallisierung von Epirubicinhydrochlorid aus dieser Mischung;
wobei die Mischung in Schritt (b) ferner Wasser enthält.

2. Verfahren nach Anspruch 1, **dadurch gekennzeichnet, dass** in Schritt (a) eine Lösung von Epirubicinhydrochlorid bereitgestellt wird.

3. Verfahren nach Anspruch 2, **dadurch gekennzeichnet, dass** eine wässrige Lösung von Epirubicinhydrochlorid bereitgestellt wird.

4. Verfahren nach Anspruch 3, **dadurch gekennzeichnet, dass** der Anteil an Epirubicinhydrochlorid 100 - 400 g/l und vorzugsweise 150 - 350 g/l, bezogen auf das Gesamtvolumen der Epirubicinhydrochlorid enthaltenden wässrigen Lösung, beträgt.

5. Verfahren nach Anspruch 1, **dadurch gekennzeichnet, dass** in Schritt (a) Epirubicinhydrochlorid in kristalliner oder amorpher Form bereitgestellt wird.

6. Verfahren nach einem der vorangegangenen Ansprüche, **dadurch gekennzeichnet, dass** der Anteil des wenigstens einen Alkohols, der aus der Gruppe ausgewählt ist, die aus 1-Butanol, 2-Butanol und 1-Pentanol besteht, im Bereich von 5 - 100 Volumenprozent, vorzugsweise im Bereich von 5 - 50 Volumenprozent und mehr bevorzugt im Bereich von 7 - 15 Volumenprozent, bezogen auf das Gesamtvolumen der Mischung in Schritt (b), liegt.

7. Verfahren nach einem der vorangegangenen Ansprüche, **dadurch gekennzeichnet, dass** die Mischung in Schritt (b) wenigstens einen weiteren Alkohol enthält, der aus der Gruppe ausgewählt ist, die aus Ethanol, 1-Propanol und 2-Propanol besteht.

8. Verfahren nach Anspruch 7, **dadurch gekennzeichnet, dass** der Anteil des wenigstens einen weiteren Alkohols, der aus der Gruppe ausgewählt ist, die aus Ethanol, 1-Propanol und 2-Propanol besteht, im Bereich von 5 - 95 Volumenprozent, bezogen auf das Gesamtvolumen der Mischung, liegt.

9. Verfahren nach Anspruch 7 oder 8, **dadurch gekennzeichnet, dass** das Verhältnis des Volumens des weiteren Alkohols, der aus der Gruppe ausgewählt ist, die aus Ethanol, 1-Propanol und 2-Propanol besteht, zu dem Volumen des Alkohols, der aus der Gruppe ausgewählt ist, die aus 1-Butanol, 2-Butanol und 1-Pentanol besteht, im Bereich von 3:1 bis 20:1, mehr bevorzugt im Bereich von 5:1 bis 15:1 und noch mehr bevorzugt im Bereich von 7:1 bis 10:1 liegt.

10. Verfahren nach einem der vorangegangenen Ansprüche, **dadurch gekennzeichnet, dass** der Anteil an Wasser in der Mischung im Bereich von 0,5 - 7 Volumenprozent und vorzugsweise im Bereich von 3 - 5 Volumenprozent, bezogen auf das Gesamtvolumen der Mischung, liegt.

11. Verfahren nach einem der vorangegangenen Ansprüche, **dadurch gekennzeichnet, dass** in Schritt (b) der Anteil von Epirubicinhydrochlorid im Bereich von 5 - 100 g/l, vorzugsweise im Bereich von 10 - 50 g/l und mehr bevorzugt im Bereich von 25 - 35 g/l, bezogen auf das Volumen der Mischung, liegt.

12. Verfahren nach einem der vorangegangenen Ansprüche, **dadurch gekennzeichnet, dass** der pH-Wert der Mischung in Schritt (b) im Bereich von 2,5 - 4,5, vorzugsweise im Bereich von 3,0 - 4,5, mehr bevorzugt im Bereich von 3,5 - 4,5 und noch mehr bevorzugt im Bereich von 3,9 - 4,1, liegt.

13. Verfahren nach einem der vorangegangenen Ansprüche, **dadurch gekennzeichnet, dass** in Schritt (c) die Mischung auf eine Temperatur im Bereich von 50 - 75°C, vorzugsweise im Bereich von 60 - 70°C, erwärmt wird.

14. Verfahren nach einem der vorangegangenen Ansprüche, **dadurch gekennzeichnet, dass** die Mischung bei einer Temperatur im Bereich von 50 - 75°C, vorzugsweise im Bereich von 60 - 70°C, für eine Zeitdauer von 2 - 8 Stunden, vorzugsweise für eine Zeitdauer von 4 - 6 Stunden, belassen wird.

15. Verfahren nach einem der vorangegangenen Ansprüche, **dadurch gekennzeichnet, dass** die erhaltenen Kristalle von der übrigen Mischung abgetrennt werden.

## Claims

1. A method of producing crystalline epirubicin hydrochloride comprising the steps of
(a) providing epirubicin hydrochloride,
(b) producing a mixture containing the provided epirubicin hydrochloride and at least one alcohol selected from the group consisting of 1-butanol, 2-butanol and 1-pentanol and
(c) crystallizing epirubicin hydrochloride from said mixture;
wherein the mixture of step (b) further contains water.

2. Method according to claim 1 **characterized in that** a solution of epirubicin hydrochloride is provided in step (a).

3. Method according to claim 2 **characterized in that** an aqueous solution of epirubicin hydrochloride is provided.

4. Method according to claim 3 **characterized in that** the portion of epirubicin hydrochloride is 100 - 400 g/l and preferably 150 - 350 g/l, relative to the total volume of the aqueous solution containing epirubicin hydrochloride.

5. Method according to claim 1 **characterized in that** epirubicin hydrochloride is provided in crystalline or amorphous form in step (a).

6. Method according to any of the preceding claims **characterized in that** the portion of the at least one alcohol selected from the group consisting of 1-butanol, 2-butanol and 1-pentanol is within the range of 5 - 100 per cent by volume, preferably within the range of 5 - 50 per cent by volume and more preferably within the range of 7 - 15 per cent by volume, relative to the total volume of the mixture in step (b):

7. Method according to any of the preceding claims **characterized in that** the mixture in step (b) contains at least one further alcohol selected from the group consisting of ethanol, 1-propanol and 2-propanol.

8. Method according to claim 7 **characterized in that** the portion of the at least one further alcohol selected from the group consisting of ethanol, 1-propanol, and 2-propanol is within the range of 5 - 95 per cent by volume, relative to the total volume of the mixture.

9. Method according to claim 7 or 8 **characterized in that** the ratio of the volume of the further alcohol, selected from the group consisting of ethanol, 1-propanol, and 2-propanol to the volume of the alcohol, selected from the group consisting of 1-butanol, 2-butanol, and 1-pentanol is within the range of 3:1 to 20:1, more preferably within the range of 5:1 to 15:1 and even more preferably within the range of 7:1 to 10:1.

10. Method according to to any of the preceding claims **characterized in that** the portion of water in the mixture is within the range of 0.5 - 7 per cent by volume and preferably within the range of 3 - 5 per cent by volume, relative to the total volume of the mixture.

11. Method according to any of the preceding claims **characterized in that** the portion of epirubicin hydrochloride in step (b) is within the range of 5 - 100 g/l, preferably within the range of 10 - 50 g/l and more preferably within the range of 25 - 35 g/l, relative to the volume of the mixture.

12. Method according to any of the preceding claims **characterized in that** the pH of the mixture in step (b) is within the range of 2.5 - 4.5, preferably within the range of 3.0 - 4.5, more preferably within the range of 3.5 - 4.5 and even more preferably within the range of 3.9 - 4.1.

13. Method according to any of the preceding claims **characterized in that** the mixture in step (c) is heated to a temperature within the range of 50 - 75°C, preferably within the range of 60 - 70°C.

14. Method according to any of the preceding claims **characterized in that** the mixture is kept at a temperature within the range of 50 - 75°C, preferably within the range of 60 - 70°C, over a duration of 2 - 8 hours, preferably over a duration of 4 - 6 hours.

15. Method according to any of the preceding claims **characterized in that** the obtained crystals are separated from the residual mixture.

## Revendications

1. Procédé de préparation du chlorhydrate d'épirubicine cristallin, comprenant les étapes consistant à :
(a) mettre à disposition le chlorhydrate d'épirubicine,
(b) préparer un mélange qui contient du chlorhydrate d'épirubicine et au moins un alcool choisi dans le groupe constitué par le 1-butanol, le 2-butanol et le 1-pentanol, et
(c) cristalliser le chlorhydrate d'épirubicine à partir de ce mélange ;
le mélange contenant en outre de l'eau à l'étape (b).

2. Procédé selon la revendication 1, **caractérisé en ce que**, à l'étape (a), une solution de chlorhydrate d'épirubicine est mise à disposition.

3. Procédé selon la revendication 2, **caractérisé en ce qu'**une solution aqueuse de chlorhydrate d'épirubicine est mise à disposition.

4. Procédé selon la revendication 3, **caractérisé en ce que** la proportion de chlorhydrate d'épirubicine atteint 100 à 400 g/litre et de préférence 150 à 350 g/litre, par rapport au volume total de la solution aqueuse contenant du chlorhydrate d'épirubicine.

5. Procédé selon la revendication 1, **caractérisé en ce que**, à l'étape (a), du chlorhydrate d'épirubicine est mise à disposition sous forme cristalline ou amorphe.

6. Procédé selon l'une des revendications précédentes, **caractérisé en ce que** la proportion du au moins un alcool choisi dans le groupe constitué par le 1-butanol, le 2-butanol et le 1-pentanol se situe dans la plage de 5 à 100 % en volume, de préférence dans la plage de 5 à 50 % en volume et plus préférentiellement dans la plage de 7 à 15 % en volume, par rapport au volume total du mélange à l'étape (b).

7. Procédé selon l'une des revendications précédentes, **caractérisé en ce que** le mélange, à l'étape (b), contient au moins un autre alcool, qui est choisi dans le groupe constitué par l'éthanol, le 1-propanol et le 2-propanol.

8. Procédé selon la revendication 7, **caractérisé en ce que** la proportion du au moins un autre alcool choisi dans le groupe constitué par l'éthanol, le 1-propanol et le 2-propanol se situe dans la plage de 5 à 95 % en volume, par rapport au volume total du mélange.

9. Procédé selon la revendication 7 ou 8, **caractérisé en ce que** le rapport des volumes entre l'autre alcool choisi dans le groupe constitué par l'éthanol, le 1-propanol et le 2-propanol et l'alcool choisi dans le groupe constitué par le 1-butanol, le 2-butanol et le 1-pentanol se situe dans la plage de 3 : 1 à 20 : 1, plus préférentiellement dans la plage de 5 : 1 à 15 : 1 et encore plus préférentiellement dans la plage de 7 : 1 à 10 : 1.

10. Procédé selon l'une des revendications précédentes, **caractérisé en ce que** la proportion d'eau dans le mélange se situe dans la plage de 0,5 à 7 % en volume et de préférence dans la plage de 3 à 5 % en volume, par rapport au volume total du mélange.

11. Procédé selon l'une des revendications précédentes, **caractérisé en ce que**, à l'étape (b), la proportion de chlorhydrate d'épirubicine se situe dans la plage de 5 à 100 g/litre, de préférence dans la plage 10 à 50 g/litre et plus préférentiellement dans la plage de 25 à 35 g/litre, par rapport au volume du mélange.

12. Procédé selon l'une des revendications précédentes, **caractérisé en ce que** le pH du mélange, à l'étape (b), se situe dans la plage de 2,5 à 4,5, de préférence dans la plage de 3,0 à 4,5, plus préférentiellement dans la plage de 3,5 à 4,5 et encore plus préférentiellement dans la plage de 3,9 à 4,1.

13. Procédé selon l'une des revendications précédentes, **caractérisé en ce que**, à l'étape (c), le mélange est chauffé à une température située dans la plage de 50 à 75°C, de préférence dans la plage de 60 à 70°C.

14. Procédé selon l'une des revendications précédentes, **caractérisé en ce que** le mélange est laissé à une température située dans la plage de 50 à 75°C, de préférence dans la plage de 60 à 70°C, pendant une durée de 2 à 8 heures, de préférence pendant une durée de 4 à 6 heures.

15. Procédé selon l'une des revendications précédentes, **caractérisé en ce que** les cristaux obtenus sont séparés du reste du mélange.
